# EUROPEAN PATENT SPECIFICATION

(11) **EP 0 888 766 B1**
(45) Date of publication and mention of the grant of the patent: **09.01.2002**
(21) Application number: 98305141.8
(22) Date of filing: 29.06.1998
(51) Int. Cl.: A61F 13/15, A61F 13/40

(54) **Disposable nappy**
Wegwerfwindel
Couche à jeter

(30) Priority: 30.06.1997 GB 9713793
(43) Date of publication of application: 07.01.1999
(73) Proprietor: YKK CORPORATION, Chiyoda-ku, Tokyo (JP)
(72) Inventor: Sakai, Yuichi, London N12 7DW (GB)
(74) Representative: Luckhurst, Anthony Henry William

(56) References cited:
- EP-A- 0 496 716
- EP-A- 0 875 226
- DE-A- 3 121 390
- GB-A- 2 060 398
- US-A- 4 604 096

## Description

### BACKGROUND OF THE INVENTION

### 1. Field of the Invention

The present invention relates to a disposable nappy, and more particularly, it relates to a disposable nappy having features convenient for wrapping after it is used.

### 2. Description of the Related Art

The disposal of disposable nappies presents a problem for the user. Users adopt various techniques for folding or rolling up the used or soiled nappy and utilize the fastening tapes for securing the folded/rolled nappy in a closed position. The nappy might then be disposed of if a waste receptacle is to hand, or it might be carried by a user until a receptacle is found. In the latter event, the user will often place the nappy in a paper or plastic bag carried for the purpose.

The traditional touch sensitive adhesive based fastening tapes for nappies have been developed to the point where the tape can be re-used once or twice for re-securing the nappy. However, some systems require a special tape receiving area on the nappy and so the tape does not work well when used to secure a folded or rolled, used nappy. Systems based on hook and loop fasteners have also been developed. EP-A-0 321 234 describes a system in which an additional landing area is provided to receive the fastener when the nappy is folded or rolled for disposal.

US-A-4 604 096 describes a nappy with an "integral disposal system" in the form of an envelope or bag which is attached to the nappy. This system requires a strap which holds the bag snug against the nappy while it is being used, and can then be used to close the bag after the soiled nappy has been folded and placed in the bag.

The system disclosed in EP-A-0 321 234 requires the use of an additional fastener component - to provide the landing area, and also requires the user to learn to fold or roll the nappy in a particular way. With the "integral disposal system" disclosed in US-A-4 604 096, the use of a long strap for holding the bag in place on the wearer is unsightly, inconvenient and adds to the expense of the system.

EP-A-496 716 describes a disposable nappy with a pocket formed on an outer surface of the nappy. The pocket extends over the crotch region of the nappy and the mouth of the pocket faces the waist region of the nappy. After use, the nappy is folded at the crotch region only and the pocket then inverted.

EP-A-875 226 which was published after the priority date of the present application describes a pull on garment such as a nappy with a downwardly opening pocket on the front of the nappy.

### SUMMARY OF THE INVENTION

The present invention provides a disposable nappy having a body comprising front and back regions separated by a crotch region and wings on the front and back regions, the wings being overlapped for securing the nappy on a wearer, and a pocket on the outer surface of the body the pocket being invertable to contain within it the body of the nappy, characterised in that the pocket is provided on the front or back region and the mouth of the pocket faces the crotch region.

The soiled nappy is folded to bring ends of a front and a back and crotch region of the nappy into a compact configuration, with the pocket exposed, the pocket is then inverted, i.e. turned inside out, so that the body is then contained within the (inverted) pocket.

The pocket is held substantially flat against the outer surface of the nappy, so that the pocket is not noticeable and is neat in appearance.

One major wall of the pocket may be fixed substantially fully against the outer surface of the nappy to hold the pocket flat against the outer surface.

Very preferably the pocket is formed by a panel on the outer surface of the nappy, the panel and the outer surface forming the walls of the pocket. Usually, the material of the panel is the same as the one used for the layer of the nappy.

Preferably the panel is secured to the outer surface around an edge region of the panel, except for a length of the edge region which forms a mouth of the pocket. A releasable fastening, such as an adhesive, may be used to secure the mouth region closed while the nappy is in use. However, by securing the panel flat against the outer surface, it may not be necessary to secure the mouth closed.

Preferably the panel is a solid sheet, to enhance the visual effect, but an open mesh or strips could be used to form the pocket, or the panel.

### BRIEF DESCRIPTION OF THE DRAWINGS

Figure 1 is a rear view of a nappy opened out, according to a typical embodiment of the invention;
Figure 2 is a perspective view of the nappy of Figure 1, folded about a crotch region;
Figure 3 is a cross-section along the line III-III of Figure 2;
Figure 4 is a perspective view showing a used nappy in a folded condition; and
Figure 5 is a perspective view showing the nappy of Figure 4 with the pocket inverted.

### DETAILED DESCRIPTION OF THE EMBODIMENTS

The invention will be further described by way of example with reference to the accompanying drawings.

The figures show a disposable nappy. The construction of such nappies is well known in the art and is shown only schematically here.

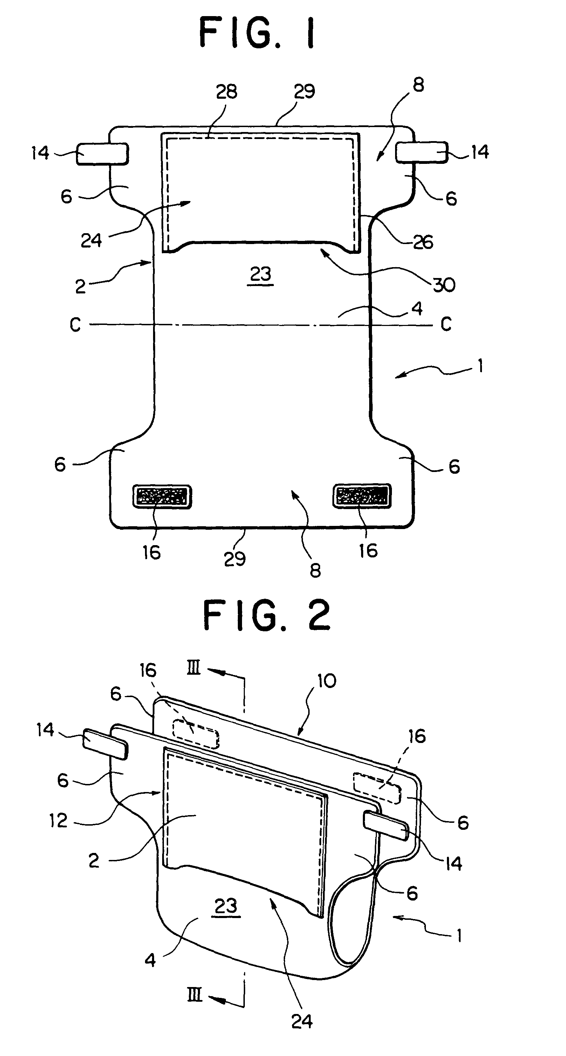

Figure 1 shows a plan view of a disposable nappy, opened out. The nappy body 1 is of typical construction having a waisted body 2 with a crotch region 4 which fits between the legs of a wearer, and wings 6 formed on ends 8 of the body. The wasted body 2 forms front 10 and back 12 of the nappy when worn (see Figure 2), with the wings 6 being overlapped for securing the nappy on a wearer. The nappy of this embodiment has hook and loop fasteners to secure the wings 6 in place, with hook fastener portions 14 on the wings 6 of the back 12 of the nappy, and loop portions 16 on the front 10 of the nappy.

The nappy body 1 is of three layers construction with a liquid impervious outer layer 22, a pervious inner layer 18, and an absorbent material 20 between the layers.

The construction thus far described is well known.

Referring to Figures 1 to 3, a pocket 24 is formed on the outer surface 23 of the outer layer 22 in the back 12 by a panel 26 of sheet material. Conveniently the material of the panel 26 is the same as the material of the outer layer 22, but the other material could be used. For example, a mesh material or an open construction using overlapping tapes could be used to form the pocket 24.

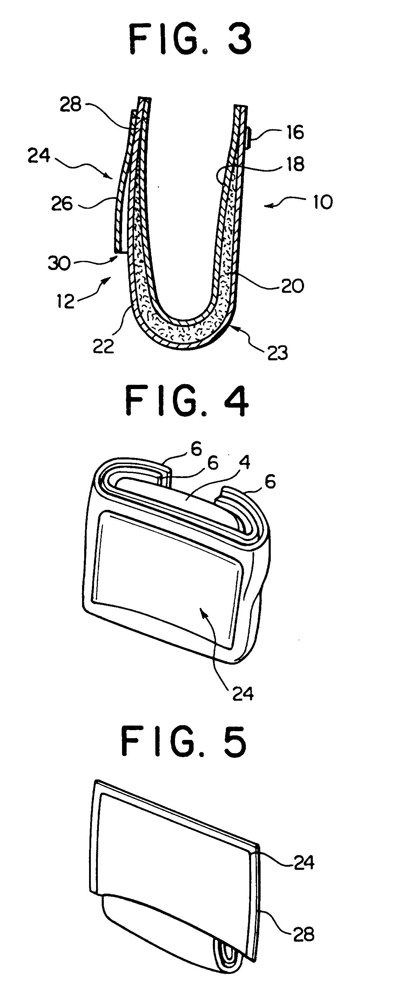

The panel 26 is rectangular and secured to the outer layer 22 by gluing or welding along three sides, at an edge region 28, the fourth side forming a mouth 30 of the pocket (Figure 3). The panel 26 preferably extends over about one third of the nappy measured between the edges 29.

Figure 4 shows a used and folded nappy, and Figure 5 shows an example in which the nappy body 1 is contained in the pocket 24 of Figure 4 being inverted.

The used nappy may be folded or rolled in various ways for disposal. The pocket 24 is arranged to be on the outside of the folded nappy, and the pocket 24 is then inverted so that the inverted pocket 24 contains the folded nappy.

In Figure 4, the nappy has been folded about a centreline C (Figure 1), it is then folded again to bring the crotch region 4 opposite the front 10, and the wings 6 are then folded around the crotch region 4. It will be appreciated that other folding arrangements are possible.

The pocket 24 could be provided on another region of the outer surface 23 of the outer layer 22. For example, the pocket 24 could be provided on the front 10.

Also, the material of the panel 26 may be elasticised to facilitate inversion of the pocket.

Other modifications will be apparent to those in the art and it is desired to include all such modifications as fall within the scope of the accompanying claims.

## Claims

1. A disposable nappy having a body (1) comprising front and back regions (10, 12) separated by a crotch region (4) and wings (6) on the front and back regions (10, 12), the wings being overlapped for securing the nappy on a wearer, and a pocket (24) on the outer surface (23) of the body (1), the pocket being provided on the front or back region (10,12) and invertable to contain within it the body (1) of the nappy, **characterised in that** the mouth (30) of the pocket faces the crotch region (4).

2. A disposable nappy as claimed in claim 1, **characterised in that** the pocket is provided on the back region (12) of the body (1).

3. A nappy as claimed in claim 1 or 2, wherein the pocket (24) is held substantially flat against the outer surface (23) of the body (1) of the nappy.

4. A nappy as claimed in claim 3, wherein one major wall of the pocket (24) is fixed substantially fully against the outer surface (23) of the nappy to hold the pocket (24) flat against the outer surface (23).

5. A nappy as claimed in claim 1, 2 or 3, wherein the pocket (24) is formed by a panel (26) on the outer surface (23) of the body (1) of the nappy, the panel (26) and the outer surface (23) forming walls of the pocket (24).

6. A nappy as claimed in claim 5, wherein the panel (26) is secured to the outer surface (23) around an edge region (28) of the panel (26), except for a length of the edge region (28) which forms a mouth (30) of the pocket (24).

7. A nappy as claimed in claim 5 or 6, wherein the panel (26) is formed of a solid sheet of material.

8. A disposable nappy as claimed in any one of the preceding claims, **characterised in that** in use the soiled nappy is folded by folding about a centre line to lay the front region (10) against the back region (12), folding the crotch (4) region against the front or back region (10, 12), wrapping the wings (6) around the folded crotch region (4), and then inverting the pocket (24).

## Patentansprüche

1. Wegwerfwindel mit einem Körper (1), welcher vordere und hintere Bereiche (10, 12), die durch einen Schrittbereich (4) getrennt sind, und an den vorderen und hinteren Bereichen (10, 12) befindliche Flügel (6), wobei die Flügel überlappt werden, um die Windel an einer die Windel tragenden Person zu befestigen, sowie eine Tasche (24) auf der Außenfläche (23) des Körpers (1) aufweist, wobei die Tasche am vorderen oder am hinteren Bereich (10, 12) vorgesehen ist und umgewendet werden kann, um den Körper (1) der Windel unterzubringen, **dadurch gekennzeichnet, dass** die Öffnung (30) der Tasche dem Schrittbereich (4) zugewandt ist.

2. Wegwerfwindel nach Anspruch 1, **dadurch gekennzeichnet, dass** die Tasche am hinteren Bereich (12) des Körpers (1) vorgesehen ist.

3. Wegwerfwindel nach Anspruch 1 oder 2, bei welcher die Tasche (24) im Wesentlichen flach gegen die Außenfläche (23) des Körpers (1) der Windel gehalten wird.

4. Wegwerfwindel nach Anspruch 3, bei welcher eine Hauptwandung der Tasche (24) im Wesentlichen vollständig an der Außenfläche (23) der Windel befestigt ist, um die Tasche (24) flach gegen die Außenfläche (23) zu halten.

5. Wegwerfwindel nach Anspruch 1, 2 oder 3, bei welcher die Tasche (24) auf der Außenfläche (23) des Körpers (1) der Windel aus einer Tafel (26) ausgebildet ist, wobei die Tafel (26) und die Außenfläche (23) Wandungen der Tasche (24) bilden.

6. Wegwerfwindel nach Anspruch 5, bei welcher die Tafel (26) an der Außenfläche (23) entlang eines Randbereichs (28) der Tafel (26) befestigt ist, außer auf einer Länge des Randbereichs (28), welche die Öffnung (30) der Tasche (24) bildet.

7. Wegwerfwindel nach Anspruch 5 oder 6, bei welcher die Tafel (26) aus einer vollwandigen Materialschicht ausgebildet ist.

8. Wegwerfwindel nach einem der vorstehenden Ansprüche, **dadurch gekennzeichnet, dass** bei Gebrauch die verschmutzte Windel gefaltet wird, indem sie an einer Mittellinie gefaltet wird, so dass der vordere Bereich (10) auf dem hinteren Bereich (12) zu liegen kommt, der Schrittbereich (4) gegen den vorderen oder den hinteren Bereich (10, 12) gefaltet wird, die Flügel (6) um den gefalteten Schrittbereich (4) gewickelt werden und dann die Tasche (24) umgewendet wird.

## Revendications

1. Couche jetable ayant un corps (1) qui comprend des régions avant et arrière (10, 12) séparées par une région d'entrejambe (4) et des rabats (6) sur les régions avant et arrière (10, 12), les rabats se chevauchant pour fixer la couche sur un porteur, et une poche (24) sur la surface extérieure (23) du corps (1), la poche étant prévue sur la région avant ou arrière (10, 12) et réversible pour contenir à l'intérieur le corps (1) de la couche, **caractérisée en ce que** l'ouverture (30) de la poche est tournée vers la région d'entrejambe (4).

2. Couche jetable selon la revendication 1, **caractérisée en ce que** la poche est prévue sur la région arrière (12) du corps (1).

3. Couche selon la revendication 1 ou 2, dans laquelle la poche (24) est maintenue sensiblement à plat contre la surface extérieure (23) du corps (1) de la couche.

4. Couche selon la revendication 3, dans laquelle une paroi principale de la poche (24) est fixée sensiblement complètement contre la surface extérieure (23) de la couche pour maintenir la poche (24) à plat contre la surface extérieure (23).

5. Couche selon la revendication 1, 2 ou 3, dans laquelle la poche (24) est formée par un panneau (26) sur la surface extérieure (23) du corps (1) de la couche, le panneau (26) et la surface extérieure (23) formant les parois de la poche (24).

6. Couche selon la revendication 5, dans laquelle le panneau (26) est fixé à la surface extérieure (23) autour d'une région de bord (28) du panneau (26), sauf sur une longueur de la région de bord (28) qui forme une ouverture (30) de la poche (24).

7. Couche selon la revendication 5 ou 6, dans laquelle le panneau (26) est constitué d'une feuille de matériau plein.

8. Couche jetable selon l'une quelconque des revendications précédentes, **caractérisée en ce que**, en utilisation, la couche souillée est pliée en la pliant autour d'un axe pour appliquer la région avant (10) contre la région arrière (12), en pliant la région d'entrejambe (4) contre la région avant ou arrière (10, 12), en enveloppant la région d'entrejambe (4) avec les rabats (6), puis en retournant la poche (24).
